Europäisches Patentamt

European Patent Office

Office européen des brevets

(11) Publication number: **0 396 203**
**A2**

(12) **EUROPEAN PATENT APPLICATION**

(21) Application number: **90201121.2**

(22) Date of filing: **03.05.90**

(51) Int. Cl.⁵: **C08G 59/32, C08G 59/04**

(30) Priority: **05.05.89 US 347690**

(43) Date of publication of application:
**07.11.90 Bulletin 90/45**

(84) Designated Contracting States:
**DE NL**

(71) Applicant: SHELL INTERNATIONALE
RESEARCH MAATSCHAPPIJ B.V.
Carel van Bylandtlaan 30
NL-2596 HR Den Haag(NL)

(72) Inventor: **Bauer, Ronald Sherman**
**5427 Crown Colony**
**Houston, Texas 77069(US)**

(74) Representative: **Aalbers, Onno et al**
**P.O. Box 302**
**NL-2501 CH The Hague(NL)**

(54) **Encapsulating semiconductors.**

(57) An epoxy resin encapsulation compositon comprising at least one filler, a tetrakisglycidyl ether of an $\alpha,\alpha,\omega,\omega$-tetrakis(hydroxyphenyl)$C_4$-$C_{14}$ alkane and a curing agent, and a method of encapsulating with the inventive composition are described.

EP 0 396 203 A2

EP 0 396 203 A2

## ENCAPSULATING SEMICONDUCTORS

This invention relates to the use of defined epoxy resin compositions for encapsulating semiconductors.

Encapsulation of semiconductors and other components is widely used in the electronics industry to provide physical integrity and environmental protection to electrical components. Encapsulation is generally a process in which an electrical component, or an assembly of small discrete units, is cast with, or embedded in, a molten film, sheath, or foam of an encapsulation composition, which is then subjected to conditions effective to solidify the composition. Once the encapsulation composition is solidified, the encapsulated component will have good physical integrity and environmental protection. The most commonly used encapsulation technique is transfer moulding. In this process, the component to be encapsulated is placed into the cavity of a mould and an encapsulation composition, liquefied by heat and pressure, is forced into the cavity where it solidifies giving the familiar encapsulated device.

An encapsulation composition must not soften during the normal usage of the encapsulated component, must not develop excessive internal stress during the encapsulated process, and must have minimal moisture gain. An encapsulation composition that softens during the normal usage of the encapsulated component will jeopardize the integrity of the encapsulated component. The softening of the encapsulation composition during the normal usage of the encapsulated component can be prevented by incorporating into the composition an encapsulation resin with a sufficiently high glass transition temperature ($T_g$). Encapsulation compositions that develop high internal stress tend to break apart during normal usage. During soldering processes, an encapsulated component is subjected to very high temperatures. Those encapsulation compositions with high moisture contents will tend to break apart during the soldering process as the moisture is driven off.

Conventional epoxy resins that are used in encapsulation compositions generally have, when cured, a sufficiently high $T_g$, generally at least 150 °C for encapsulation, but are generally deficient in resistance to internal stress, a function of the elastic modulus of the composition. Generally the $T_g$ and the elastic modulus vary proportionally with each other. That is, the reduction of the elastic modulus will also result in reduction of the $T_g$. Often a reduction of the elastic modulus that reduces the internal stress to a suitable level, will result in too great a reduction of the $T_g$ of the encapsulation resin, thus resulting in an encapsulation composition that will soften during the normal usage of the encapsulated component.

It is therefore an object of the present invention to provide an epoxy encapsulation composition which has both high $T_g$ and an attractive flexural modulus, i.e. a modulus below 70 kg.cm$^{-2}$.

According to the invention, there is provided the use for encapsulating semi-conductors of a composition comprising from 15 to 30 %wt of a tetrakisglycidyl ether of an $\alpha,\alpha,\omega,\omega$-tetrakis(4-hydroxyphenyl)C$_4$-C$_{14}$ alkane and from 85 to 70 %wt of a curing agent for the tetrakisglycidyl ether.

The encapsulation compositions used in this invention comprise a tetrakisglycidyl ether of a tetraphenol. To provide for the desired physical properties for encapsulation, the resin component of the encapsulation composition will have when cured a $T_g$ greater than 150 °C, preferably greater than 170 °C, most preferably greater than 180 °C, and a flexural modulus less than 70 kg.cm$^{-2}$, preferably less than 68 kg.cm$^{-2}$, and most preferably less than 65 kg.cm$^{-2}$.

The aliphatic hydrocarbon chain in the alkane constituent of the tetrakisglycidyl ethers generally contains from 4 carbon atoms to 14 carbon atoms, preferably from 8 to 12 carbon atoms, and most preferably 12 carbon atoms. The 4-hydroxyphenyl groups may be substituted or unsubstituted in the 3- and/or 5-position. A variety of tetraphenols can be used, although it is preferred to employ 1,1,5,5-tetrakis(4-hydroxy-3,5-dimethylphenyl)pentane, 1,1,8,8-tetrakis(4-hydroxy-3-methylphenyl)octane, 1,1,8,8-tetrakis(4-hydroxyphenyl)octane, 1,1,12,12-tetrakis(4-hydroxy-3-methylphenyl)dodecane and 1,1,12,12-tetrakis(4-hydroxyphenyl)dodecane. The most preferred $\alpha,\alpha,\omega,\omega$-tetrakis(4-hydroxphenyl)alkanes are 1,1,2,2-tetrakis(4-hydroxy-3,5-dimethylphenyl)pentane, 1,1,8,8-tetrakis(4-hydroxyphenyl)octane, and 1,1,12,12-tetrakis(4-hydroxyphenyl)dodecane.

The suitable tetraphenols are known compounds and they can be produced by condensing the appropriate dialdehyde with the appropriate phenol. This condensation reaction is generally effected by mixing a substantial stoichiometric excess of the phenol with the dialdehyde, saturating the mixture with an acid, preferably hydrogen chloride, allowing the mixture to react, and removing the unreacted phenol and any solvent used by distillation.

Methods of producing the suitable polyglycidyl ethers are also known per se and involve reacting the appropriate tetraphenol with a halohydrin in the presence of an alkali metal hydroxide.

The encapsulation composition used in this invention includes a curing agent for the polyglycidyl ether. Suitable curing agents include amine, phenolic, novolac, anhydride and imidazole curing agents. Anhydride

2

and novolac curing agents are preferred, with novolac curing agents being the most preferred. Specific examples of novolac curing agents include Durite SD1711 (Durite is a trade mark) and Durite SD17131, made by Borden Chemical Company. A curing amount of the curing agent is used. Typically, curing is facilitated with an amount within the range of 1 to 200 weight per cent of curing agent based on the weight of encapsulation resin, preferably an amount within the range of 50 to 60 weight per cent.

Although the encapsulation composition can be comprised entirely of the encapsulation resin and curing agent, the encapsulation composition may include other materials and fillers including stabilizers, extenders, other resins, pigments, reinforcing agents, flow control agents, flame retardants, metal oxide fillers, mineral fillers and other appropriate ingredients as known in the encapsulation art. Preferably the encapsulation composition comprises a mineral filler such as silica.

The encapsulation composition will preferably contain in the range of from 200 to 500 parts by weight, per 100 parts by weight of tetrakisglycidyl ether of a silica filler.

Methods for formulating an encapsulation resin, curing agent, fillers and other materials into an encapsulation composition are well known. Generally the encapsulation composition will be formulated by first grinding the resin, curing agent, the silica filler and other materials into fine particles, typically with the use of a grinder or a hammer mill. The particles are then screened and dry blended. Next the dry blended mixture is melt mixed, in a roll mill or extruder, at a temperature that is above the softening point of the mixture, generally 15 °C to 30 °C above the softening point of the mixture. After melt mixing, the resultant melt-mixed composition is reground and rescreened. Next, preforms are made from the resultant encapsulation composition, usually at room temperature using pressure. The preforms are then ready to be cast in a moulding machine. Generally the preforms are preheated to 90 °C, usually in a radio frequency oven, and then placed in a moulding machine operating at a temperature within the range of from 160 °C to 180 °C, during which the hardening of the encapsulation composition will take place.

## EXAMPLE 1

1,1,2,2-tetrakis(4-hydroxy-3,5-dimethylphenyl)ethane was prepared as follows. Starting materials were:
1833.0 g (15 mols) 2,6-dimethylphenol
500 ml methanol
15 ml HCl
66 g 1,2-dihydroxy ethane.

The phenol and the HCl were charged to a 3000 ml round bottomed flask and heated to 70 °C while stirring. The diol was dissolved into methanol and added dropwise to the reaction mixture over an approximate two hour period. The reaction mixture was then refluxed for approximately 17 hours at 82 °C. After refluxing, the reaction mixture was cooled to 25 °C in an ice bath, and then filtered, with the filter cake washed with toluene. A roto-vap was then used to remove the remaining methanol and unreacted phenol. The yield was 188.0 g of the tetraphenol.

## EXAMPLE 2

1,1,2,2-tetrakis(4-glycidyloxy-3,5-dimethylphenyl)ethane was prepared from the product of Example 1 as follows. Starting materials were:
432 g isopropyl alcohol (IPA)
125 g water
176 g 20% NaOH aqueous solution
102 g (0.2 moles) 1,1,2,2-tetrakis(4-hydroxy-3,5-dimethylphenyl)ethane prepared as in Example 1
740.2 g (20.0 moles) epichlorohydrin (ECH).

The water, ECH, IPA and the tetraphenol were charged to a 3000 ml round bottomed flask and heated to 70 °C. 45%, 36% and 29% of the NaOH solution was added dropwise to the reaction mixture of 16, 12 and 8 minutes, respectively. After each addition, there was a hold for 4, 8 and 12 minutes respectively. After each hold, the bottom brine layer was removed. After the third brine removal the remaining IPA, ECH, and water were evaporated from the organic layer using a roto-vap. The product was then held at 150 °C for approximately 25 minutes. Methylisobutylketone was then added to the solid product until the mixture comprised 30% solids. 500 g of 5% NaOH was then charged to the reaction mixture, and it was refluxed to 87 °C for one hour. The lower water layer was removed, and the organic layer was washed four times with 400 ml of hot water each time, and the product was then dried over MgSO₄. The product was then filtered

3

and washed with heptane yielding 120 g of glycidyl ether.

EXAMPLES 3 to 23 (odd numbered)

By procedures analogous to Example 1, employing appropriate α,ω-dihydroxyalkanes and substituted or unsubstituted phenols the following compounds were obtained.

1,1,2,2-tetrakis(4-hydroxy-3-methylphenyl)ethane, starting from 1622.0 g o-cresol and 1,2-dihydroxyethane. Yield was 141.6 g (Example 3).

1,1,2,2-tetrakis(4-hydroxyphenyl)ethane, starting from 1505.8 g phenol and 1,2-dihydroxyethane. Yield was 239.6 g (Example 5).

1,1,5,5-tetrakis(4-hydroxy-3,5-dimethylphenyl)pentane, starting from 1622.0 g phenol and 1,5-dihydroxypentane. Yield was 470.6 g (Example 7).

1,1,5,5-tetrakis(4-hydroxy-3-methylphenyl)pentane, starting from 1622.0 g o-cresol and 1,5-dihydroxypentane. Yield was 470.0 g (Example 9).

1,1,5,5-tetrakis(4-hydroxyphenyl)pentane, starting from 1618.0 g phenol and 1,5-dihydroxypentane. Yield was 452.0 g (Example 11).

1,1,8,8-tetrakis(4-hydroxy-3,5-dimethylphenyl)octane, starting from 1954.7 g 2,6-dimethylphenol and 1,8-dihydroxyoctane. Yield was 458.0 g (Example 13).

1,1,8,8-tetrakis(4-hydroxy-3-methylphenyl)octane, starting from 1622.1 g o-cresol and 1,8-dihydroxyoctane. Yield was 450.0 g (Example 15).

1,1,8,8-tetrakis(4-hydroxyphenyl)octane, starting from 1411.5 g phenol and 1,8-dihydroxyoctane. Yield was 387.5 g (Example 17).

1,1,12,12-tetrakis(4-hydroxy-3,5-dimethylphenyl)dodecane, starting from 1466.0 g 2,6-dimethylphenol and 1,12-dihydroxydodecane. Yield was 447.3 g (Example 19).

1,1,12,12-tetrakis(4-hydroxy-3-methylphenyl)dodecane, starting from 1204.5 g o-cresol and 1,12-dihydroxydodecane. Yield was 434.7 g (Example 21).

1,1,12,12-tetrakis(4-hydroxyphenyl)dodecane, starting from 1411.5 g phenol and 1,12-dihydroxydodecane. Yield was 473.8 g (Example 23).

EXAMPLES 4 to 24 (even numbered)

Each of the tetraphenols referred to in the Examples listed above were converted into their respective tetraglycidoxy ethers by processing analogous to the recipe of Example 2.

The properties of the cured resins of the above Examples are summarized in the Table below. Flexural properties, fracture toughness, dielectric constant, and dissipation factor were determined using ASTM test numbers D 790-86, D 790, D 229 and D 229, respectively. $T_g$ was obtained using a Rheometrics Viscoelastic Tester. Samples are heated from 30 °C to a maximum of 300 °C in -8 °C increments with a 2 minute hold at temperature before obtaining the data. The coefficient of thermal expansion was obtained using a DuPont dynamic mechanical analyzer. The curing cycle employed was 2 hours heating at 150 °C and 4 hours at 200 °C. The curing agent was Durite SD 1711 in an amount of 85% of stoichiometric.

4

EP 0 396 203 A2

TABLE

| Example | 2 | 8 | 14 | 20 | 4 | 10 |
|---|---|---|---|---|---|---|
| $T_g$, °C | 252 | 200 | 155 | 96 | -- | 197 |
| Flexural Properties | | | | | | |
| Strength, $kg.cm^{-2}$ | 2.44 | 2.14 | 2.75 | 1.52 | 2.59 | 2.75 |
| Modulus, $kg.cm^{-2}$ | 63.9 | 66.6 | 67.6 | 69.6 | 77.7 | 72.2 |
| Elongation, % | 5.8 | 3.9 | 5.7 | 3.5 | 4.4 | 5.6 |
| Fracture Toughness $kg.cm^{-2}$ | 77 | 70.5 | 76 | 84 | 85 | 78 |
| Moisture Gain, %wt | 2.5 | 2.0 | 2.0 | 3.1 | 2.8 | 2.0 |
| Dielectric Constant, 1MHz | 3.6 | 3.6 | 3.4 | 3.3 | 3.4 | 3.5 |
| Dissipation Factor, 1MHz | 0.031 | 0.025 | 0.021 | 0.017 | 0.019 | 0.017 |
| Coef. of Thermal Expansion | | | | | | |
| $<T_g$, CTE x $10^{-6}$m/m/°C | 71 | 77 | 77 | 71 | 76 | 69 |
| $>T_g$, CTE x $10^{-6}$m/m/°C | 163 | 168 | 194 | 197 | 188 | 164 |

**TABLE (Cont'd)**

| Example | 16 | 22 | 6 | 12 | 18 | 24 |
|---|---|---|---|---|---|---|
| $T_g$, °C | 167 | 157 | 241 | 247 | 172 | 189 |
| Flexural Properties | | | | | | |
|   Strength, kg.cm$^{-2}$ | 1.98 | 2.44 | 1.98 | 2.29 | 2.29 | 1.98 |
|   Modulus, kg.cm$^{-2}$ | 63 | 57.2 | 69.2 | 68.7 | 61.5 | 60.5 |
|   Elongation, % | 4.2 | 6.8 | 3.8 | 4.7 | 4.6 | 4.3 |
| Fracture Toughness kg.cm$^{-2}$ | 81 | 84 | 69 | 76.5 | 61.5 | 74 |
| Moisture Gain, %wt | 1.9 | 1.5 | 2.7 | 2.5 | 2.1 | --- |
| Dielectric Constant, 1MHz | 3.5 | 3.2 | 3.4 | 3.3 | 3.7 | 3.8 |
| Dissipation Factor, 1MHz | 0.017 | 0.016 | 0.030 | 0.028 | 0.027 | 0.019 |
| Coef. of Thermal Expansion | | | | | | |
|   <$T_g$, CTE x 10$^{-6}$ m/m/°C | 78 | 88 | 77 | 77 | 79 | 92 |
|   >$T_g$, CTE x 10$^{-6}$ m/m/°C | 172 | 192 | 147 | 163 | 146 | 172 |

## Claims

1. The use of a composition comprising:

(a) from 15 to 30 %wt, based on the weight of the composition, of a tetrakisglycidyl ether of an $\alpha,\alpha,\omega,\omega$-tetrakis(4-hydroxyphenyl)$C_4$-$C_{14}$ alkane and

(b) from 70 to 85 %wt of a curing agent,

for encapsulating semiconductors.

2. The use of claim 1 wherein the curing agent is selected from amines, phenolics, imidazoles, anhydrides, and novolac resins.

3. The use of claim 1 wherein the composition additionally comprises from 200 to 500 parts by weigth silica per 100 parts of the tetrakisglycidyl ether.

4. The use of claim 1 wherein the tetrakisglycidyl ether is selected from 1,1,5,5,-tetrakis(4-glycidyloxy-3,5-dimethylphenyl)pentane, 1,1,8,8-tetrakis(4-glycidyloxyphenyl)octane and 1,1,12,12-tetrakis(4-glycidyloxyphenyl)dodecane.

5. The use of claim 1 wherein the cured composition has a $T_g$ of at least 170 °C and a flexural modulus of less than 65 kg.cm$^{-2}$.